# EUROPÄISCHE PATENTSCHRIFT

(11) **EP 3 844 859 B1**
(45) Veröffentlichungstag und Bekanntmachung des Hinweises auf die Patenterteilung: **05.10.2022**
(21) Anmeldenummer: 18769582.0
(22) Anmeldetag: 30.08.2018
(51) Int. Cl.: H02K 5/173, H02K 7/14, H02K 11/00, H02K 11/33, A61B 17/16, H02K 7/00, H02K 7/08

(54) **ANTRIEBSAGGREGAT FÜR EIN CHIRURGISCHES POWER-TOOL**
DRIVE UNIT FOR A SURGICAL POWER TOOL
GROUPE MOTO-PROPULSEUR POUR UN OUTIL ÉLECTRIQUE CHIRURGICAL

(43) Veröffentlichungstag der Anmeldung: 07.07.2021
(73) Patentinhaber: SMC Innovation GmbH, 6330 Cham (CH)
(72) Erfinder: JANSEN-TROY, Arne, 78333 Stockach (DE); STUDER, Armin, 6330 Cham (CH)
(74) Vertreter: Daub, Thomas
(86) Internationale Anmeldenummer: PCT/CH2018/000035
(87) Internationale Veröffentlichungsnummer: WO 2020/041907

(56) Entgegenhaltungen:
- WO-A1-2018/026735
- WO-A2-01/59288
- WO-A2-03/005529
- JP-U- S5 587 174
- US-A- 5 113 102
- US-A- 5 627 420
- US-A1- 2016 287 265

## Beschreibung

Die Erfindung bezieht sich auf ein Antriebsaggregat für ein chirurgisches Power-Tool gemäss dem Oberbegriff des Patentanspruchs 1 und auf ein Verfahren zum Aufladen der Energieversorgungseinheit des erfindungsgemässen Antriebsaggregats gemäss dem Oberbegriff des Patentanspruchs 15.

Derzeit existiert eine Vielzahl von chirurgischen Power-Tools oder Powertool-Systemen auf dem Markt wie beispielsweise Sägen, Bohrmaschinen oder Hochgeschwindigkeits-Fräser für die Orthopädie und Traumatologie. Die Verwendung von solchen chirurgischen Power-Tools im Operationssaal erfordert eine Sterilisation aller Komponenten des Power-Tools, so dass auch die mechatronischen Komponenten dem Nassdampf während der Sterilisation ausgesetzt sind.

Aus dem Dokument US 5,627,420 RINKER ET AL. ist ein eingekapselter Elektromotor bekannt, welcher eine ringförmige Statorpatrone umfasst, die als Ganzes in den Hohlraum eines Motorgehäuses eingebaut oder daraus entfernt werden kann. Für eine Reinigung oder einen Ersatz des Stators muss daher nicht der gesamte Elektromotor aus dem Motorengehäuse ausgebaut werden. Dieser bekannte Elektromotor weist jedoch den Nachteil auf, dass die Statorpatrone schwierig zu reinigen ist, weil das Gehäuse der Statorpatrone Öffnungen zur Durchführung von Elektrokabeln aufweist, um den Elektromotor an einer Energiequelle anzuschliessen.

Das Dokument US 2016/287265 A1 offenbart ein Antriebsaggregat für ein chirurgisches Power-Tool.

Hier will die Erfindung Abhilfe schaffen. Der Erfindung liegt die Aufgabe zugrunde, ein Antriebsaggregat für ein chirurgisches Power-Tool zu schaffen, welches eine einfache Reinigung und Wartung sowie eine Sterilisierung des gesamten Antriebsaggregats ermöglicht.

Die Erfindung löst die gestellte Aufgabe mit einem Antriebsaggregat für chirurgische Power-Tools, welches die Merkmale des Anspruchs 1 aufweist, sowie mit einem Verfahren zum Aufladen der Energieversorgungseinheit des erfindungsgemässen Antriebsaggregats, welches die Merkmale des Anspruchs 15 aufweist.

Die durch die Erfindung erreichten Vorteile sind im Wesentlichen darin zu sehen, dass dank des erfindungsgemässen Antriebsaggregats:
- eine vereinfachte Reinigung des gesamten Antriebsaggregats ermöglicht wird, da das gesamte Antriebsaggregat einschliesslich Energieversorgungseinheit nur zwei Baugruppen umfasst, nämlich eine Statorbaugruppe und eine Rotorbaugruppe, welche einfach voneinander demontierbar sind. Zudem wird die Reinigung auch dadurch vereinfacht, dass weder an der Statorbaugruppe noch an der Rotorbaugruppe durchgehende Öffnungen für Wellen oder zur Durchführung von Stromkabeln angebracht sind;
- durch die einfache Montier- und Demontierbarkeit der zwei Baugruppen eine vereinfachte Wartung des Antriebsaggregats ermöglicht wird. Falls ein elektronischer Defekt vorliegt, kann der Benutzer die gesamte Statorbaugruppe auf einfache Weises auswechseln; und
- eine kompakte Bauweise des Antriebsaggregats ohne elektrische Anschlüsse oder Kontakte gegen aussen ermöglicht wird.

Weitere vorteilhafte Ausgestaltungen der Erfindung können wie folgt kommentiert werden:
In einer speziellen Ausführungsform ist das Statorgehäuse mittels eines Schnellverschlusses im Gehäuse des Elektromotors (2) lösbar befestigbar. Die Wartung des Antriebsaggregats wird dadurch weiter vereinfacht, weil der Anwender in kürzester Zeit die gesamte aus Stator, Steuerung und Energieversorgungseinheit bestehende Statorbaugruppe auswechseln kann, wenn ein elektronischer Defekt auftritt. Die Wahrscheinlich eines defekten Rotors ist sehr gering, da dieser im Wesentlichen nur eine Spule umfasst.

In einer weiteren Ausführungsform umfasst der Schnellverschluss einen Druckknopf oder Schnappverschluss.

In einer anderen ausführungsform weist das Statorgehäuse ein vorderes Ende, ein hinteres Ende und einen am hinteren Ende des Statorgehäuses offenen Hohlraum auf, wobei der Hohlraum entlang der Längsachse hintereinander angeordnet einen ersten Längsabschnitt zur Aufnahme der elektrischen Statorwicklungen und einen zweiten Längsabschnitt zur Aufnahme der Energieversorgungseinheit und der Steuerung umfasst. Der dadurch resultierende Vorteil ist im Wesentlichen darin zu sehen, dass das Statorgehäuse an der Aussenseite vollständig glatt ausgebildet sein kann, so dass die Statorbaugruppe einfach zu reinigen ist.

In einer weiteren Ausführungsform ist das Statorgehäuse einstückig ausgebildet und vorzugsweise aus Kunststoff hergestellt.

In einer weiteren Ausführungsform ist der Hohlraum des Statorgehäuses mittels eines Gehäusedeckels hermetisch verschliessbar.

In einer anderen Ausführungsform umfasst das Statorgehäuse einen Gehäusedeckel, welcher mit dem Statorgehäuse vergossen oder mittels Ultraschall verschweisst ist.

Die Vorteile dieser Ausführungsformen liegen darin, dass die Statorbaugruppe gegenüber der Aussenwelt (d.h. insbesondere auch gegenüber dem Nassdampf beim Sterilisieren) vollständig abgeschirmt ist. Aggressiv wirkende Medien können nicht in die elektrischen Statorwicklungen oder in die Steuerung, respektive die Energieversorgungseinheit gelangen, so dass eine geringe Ausfallquote erreichbar ist.

In einer anderen Ausführungsform umfasst der Rotor ein Rotorgehäuse, welches eine hermetisch abgeschlossene oder abschliessbare Ummantelung der elektrischen Rotorwicklungen bildet.

In einer weiteren Ausführungsform sind das Rotorgehäuse und die Rotorachse einstückig ausgebildet.

In einer weiteren Ausführungsform umfasst das Rotorgehäuse einen Deckel, welcher am Rotorgehäuse befestigbar oder mit dem Rotorgehäuse vergossen oder verschweisst ist.

Die Vorteile dieser Ausführungsformen sind darin zu sehen, dass die Rotorbaugruppe ebenfalls gegenüber der Aussenwelt (d.h. insbesondere auch gegenüber dem Nassdampf beim Sterilisieren) vollständig abgeschirmt ist. Aggressiv wirkende Medien können nicht in die elektrischen Rotorwicklungen gelangen.

In einer anderen Ausführungsform ist der Rotor mittels Radiallagern um die Längsachse rotierbar im Gehäuse des Elektromotors gelagert.

In einer weiteren Ausführungsform sind die Radiallager Wälzlager und sind am Rotorgehäuse befestigt. Dadurch kann der Rotor zusammen mit den Radiallagern auf einfache Weise aus dem Gehäuse des Elektromotors entfernt, gereinigt und wieder eingebaut werden.

In einer weiteren Ausführungsform sind die Radiallager gekapselt. Dadurch ist der gesamte Rotor einschliesslich der Radiallager gegenüber der Aussenwelt vollständig abgeschirmt.

In einer anderen Ausführungsform umfasst das Rotorgehäuse eine zentrale Kavität, welche sich entlang der Längsachse erstreckt und zur Aufnahme des Stators konfiguriert ist. Der Rotor ist als Aussenläufer ausgebildet. Alternativ kann der Elektromotor auch so konfiguriert sein, dass der Rotor als Innenläufer ausgebildet ist und vom Stator umgeben wird.

In einer weiteren Ausführungsform umfasst die Energieversorgungseinheit einen oder mehrere wiederaufladbare Akkumulatoren, vorzugweise Lithium-Ionen-Akkumulatoren. Die Akkumulatoren sind im Statorgehäuse integriert und müssen nicht separat sterilisiert werden, so dass handelsübliche Akkumulatoren verwendet werden können.

In einer anderen Ausführungsform umfasst die Steuerung Mittel zum Umschalten zwischen einem Betriebsmodus und einem Lademodus des Antriebsaggregats. Die Akkus können - nach einer Entladung im Gebrauch des Antriebsaggregats (Betriebsmodus) - im Lademodus z.B. im Vierquadranten-Betrieb wieder aufgeladen werden, d.h. wenn ein extern getriebener rotierender Rotor (von einer Ladeeinheit) aufgesetzt wird kann Strom erzeugt werden und die Akkus geladen werden. Dazu kann die Steuerung einen Vierquadrantensteller umfassen. Vorzugsweise umfasst der Vierquadrantensteller zumindest eine elektronische H-Brückenschaltung. Die H-Brückenschaltung beinhaltet insbesondere vier Transistoren. Der Vierquadrantensteller ist dazu vorgesehen eine Gleichspannung in eine Wechselspannung variabler Frequenz und variabler Pulsbreite umzuwandeln.

In einer anderen Ausführungsform umfasst die Steuerung elektrische Mittel zum drahtlosen Empfangen von Steuerungs- und/oder Regelungssignalen. Die Steuerung kann drahtlos Signale zur Steuerung und/oder Regelung des Antriebsaggregats empfangen, so dass keine Durchgänge für elektrische Kabel am Antriebsaggregat selbst angebracht werden müssen. Das Antriebsaggregat umfasst auch keine Strom-Kontakte nach aussen. Beispielsweise kann die Steuerung Signale von einer Fernbedienung oder von einer am Antriebsaggregat angeordneten Bedieneinrichtung empfangen.

In einer weiteren Ausführungsform umfasst das Antriebsaggregat mindestens eine Bedieneinrichtung zur drahtlosen Steuerung und/oder Regelung der Steuerung. Die Bedieneinrichtung kann lösbar an der Aussenseite des Gehäuses des Elektromotors angeordnet sein, oder im Gehäuse eingelassen und umspritzt sein.

In einer anderen Ausführungsform ist das Antriebsaggregat in eine chirurgische Bohr-, Säge- oder Fräsvorrichtung einführbar oder an eine solche Vorrichtung ankoppelbar.

Auf eine weitere Kupplung (mechanisch, magnetisch etc.), welche sich ansonsten zusätzlich auf der Antriebswelle befinden müsste, kann verzichtet werden, da Achsversatz und demzufolge wirkende Zwangskräfte ausgeglichen werden können. Der Aufbau des Elektromotors als solches kann Abweichungen von der idealen Achse (Winkelfehler und Abweichungen von der Koaxialität) ausgleichen. Dies insbesondere weil der Rotor an sich direkt mit dem Abtrieb (Zahnrad für Säge oder Spindel für Bohrer oder Fräser etc.) verbunden wird. Im allgemeinen Maschinenbau übliche, dem Elektromotor seriell nachgeschaltete Kupplungen, um die Achsdifferenzen auszugleichen und somit Zwangskräfte zu verhindern, welche die Lagerstandzeit herabsetzen würden, entfallen somit. Das rotierende magnetische Feld zwischen Stator und Rotor muss nicht genau koaxial ausgerichtet sein.

Die Erfindung und Weiterbildungen der Erfindung werden im Folgenden anhand der teilweise schematischen Darstellungen eines Ausführungsbeispiels noch näher erläutert.

Es zeigen:
Fig. 1 einen Längsschnitt durch eine Ausführungsform des erfindungsgemässen Antriebsaggregats;
Fig. 2 einen Längsschnitt durch die Rotorbaugruppe der in Fig. 1 dargestellten Ausführungsform des erfindungsgemässen Antriebsaggregats; und
Fig. 3 einen Längsschnitt durch die Statorbaugruppe der in Fig. 1 dargestellten Ausführungsform des erfindungsgemässen Antriebsaggregats.

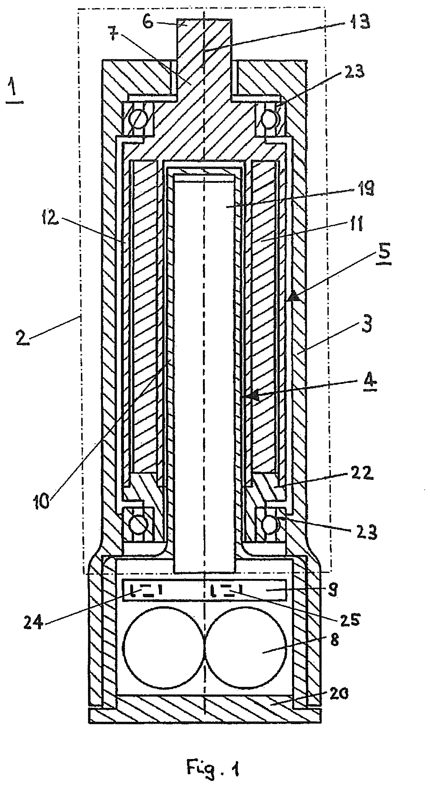

Die in den Fig. 1 - 3 dargestellte Ausführungsform des erfindungsgemässen Antriebsaggregats 1 für ein chirurgisches Power-Tool umfasst im Wesentlichen einen Elektromotor 2 mit einem Gehäuse 3, einem Stator 4 und einem Rotor 5 sowie eine Steuerung 9 für den Elektromotor 2 und eine Energieversorgungseinheit 8 für den Elektromotor 2 und die Steuerung 9. Das Antriebsaggregat 1 und insbesondere das Gehäuse 3 des Elektromotors 2 sind so konzipiert, dass das gesamte Antriebsaggregat 1 mindestens im Bereich des Elektromotors 2 in eine chirurgische Bohr-, Säge- oder Fräsvorrichtung einführbar ist oder an eine solche Vorrichtung ankoppelbar ist. Dazu umfasst der Rotor 5 eine Rotorachse 7, welche sich koaxial zur Längsachse 13 des Elektromotors 2 erstreckt und ein freies Ende 6 aufweist, wobei an diesem freien Ende 6 der Rotorachse 7 ein chirurgisches Werkzeug oder eine ein Getriebe aufweisende chirurgische Vorrichtung ankuppelbar ist.

Der Stator 4 umfasst ein hermetisch abgeschlossenes Statorgehäuse 10 und ist als Ganzes aus dem Elektromotor 2 ausbaubar, respektive in diesen einbaubar ist. Ferner sind im Statorgehäuse 10 die Energieversorgungseinheit 8 und die Steuerung 9 angeordnet.

Der Rotor 5 umfasst neben der Rotorachse 7 ein Rotorgehäuse 21 und elektrische Rotorwicklungen 11, welche in einer durch das Rotorgehäuse 21 gebildeten Ummantelung 12 hermetisch abgeschlossen angeordnet sind. Das Rotorgehäuse 21 weist ein erstes Ende 27 und ein zweites Ende 28 auf, wobei die Rotorachse 7 am ersten Ende 27 des Rotorgehäuses 21 angeordnet ist.

In der in den Fig. 1 - 3 dargestellten Ausführungsform ist der Rotor 5 beispielhaft, und nicht einschränkend, als Aussenläufer konfiguriert. Dazu umfasst das Rotorgehäuse 21 eine zentrale Kavität 26, welche sich koaxial zur Längsachse 13 erstreckt, am zweiten Ende 28 des Rotorgehäuses 21 offen ist und zur Aufnahme des Stators 4 konfiguriert ist. In einer alternativen Ausführungsform kann der Elektromotor 2 auch so konfiguriert sein, dass der Rotor 5 als Innenläufer ausgebildet ist, welcher koaxial zur Längsachse 13 in einem Hohlraum des Stators 4 angeordnet ist.

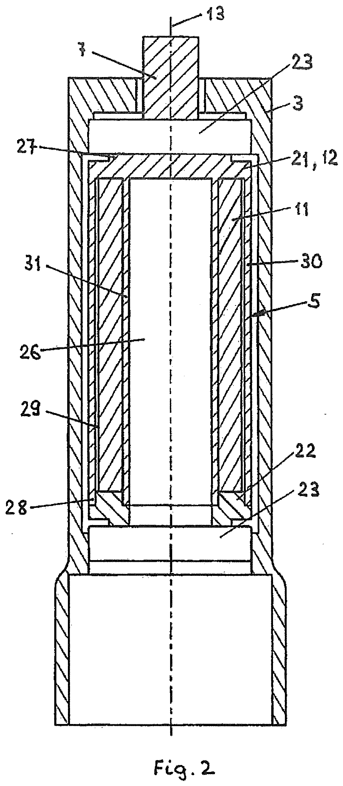

Das Rotorgehäuse 21 und die Rotorachse 7 sind beispielhaft einstückig ausgebildet und aus Kunststoff hergestellt. Zudem umfasst das Rotorgehäuse 21 einen Deckel 22, welcher mit dem Rotorgehäuse 21 vergossen oder verschweisst (z.B. mittels Ultraschall) ist. Das Rotorgehäuse 21 weist somit einen geschlossenen hohlzylindrischen Innenraum 29 auf (Fig. 2), in welchem die elektrischen Rotorwicklungen 11 eingeschlossen sind. Dieser Innenraum 29 ist gegenüber dem Gehäuse 3 des Elektromotors 2 durch eine Aussenwand 30 des Rotorgehäuses 21 und gegenüber der Kavität 26 durch eine Innenwand 31 des Rotorgehäuses 21 abgegrenzt. Ferner ist der Innenraum 29 für die elektrischen Rotorwicklungen 11 am ersten Ende 27 des Rotorgehäuses 21 geschlossen und am zweiten Ende 28 durch den Deckel 22 verschlossen.

Ferner ist der Rotor 5 mittels Radiallagern 23 um die Längsachse 13 rotierbar im Gehäuse 3 des Elektromotors 2 gelagert, wobei die Radiallager 23 beispielhaft, und nicht einschränkend, Wälzlager sind, welche am Rotorgehäuse 21 befestigt sind. Der Rotor bildet somit zusammen mit den Radiallagern eine Rotorbaugruppe, welche auf einfache Weise aus dem Gehäuse 3 des Elektromotors 2 entfernt, gereinigt und wieder eingebaut werden. Die Radiallager 23 sind gekapselt, so dass die gesamte Rotorbaugruppe gegenüber der Aussenwelt vollständig abgeschirmt ist.

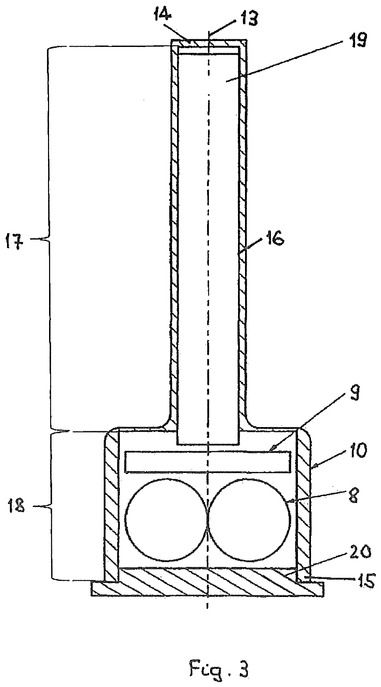

Das Statorgehäuse 10 hat ein vorderes Ende 14, ein hinteres Ende 15 und einen am hinteren Ende 15 des Statorgehäuses 10 offenen Hohlraum 16 (Fig. 3). Dieser Hohlraum 16 weist entlang der Längsachse 13 hintereinander angeordnet einen ersten zylindrischen Längsabschnitt 17 zur Aufnahme der elektrischen Statorwicklungen 19 und einen zweiten Längsabschnitt 18 zur Aufnahme der Energieversorgungseinheit 8 und der Steuerung 9 auf. Aussen ist das Statorgehäuse 10 im Bereich des ersten Längsabschnitts 17 zylindrisch ausgebildet, so dass dieser Bereich des Statorgehäuses 10 in die Kavität 26 des Rotorgehäuses 21 einführbar ist. Im Bereich des zweiten Längsabschnitts 18 ist das Statorgehäuse 10 einschliesslich des Hohlraums 16 erweitert ausgebildet, so dass die Energieversorgungseinheit 8 und die Steuerung 9 im zweiten Längsabschnitt 18 des Hohlraums 16 untergebracht werden können. Die Aussenseite des Statorgehäuses 10 ist vollständig glatt ausgebildet.

Das Statorgehäuse 10 ist beispielhaft, und nicht einschränkend einstückig ausgebildet und aus Kunststoff hergestellt. Der Hohlraum 16 des Statorgehäuses 10 ist am hinteren Ende 15 des Statorgehäuses 10 mittels eines Gehäusedeckels 20 hermetisch verschlossen, wobei der Gehäusedeckel 20 mit dem Statorgehäuse 10 vergossen oder mittels Ultraschall verschweisst ist. Dadurch wird eine Statorbaugruppe gebildet, welche gegenüber der Aussenwelt vollständig abgeschirmt ist.

Ferner ist das Statorgehäuse 10 mittels eines Schnellverschlusses (nicht gezeichnet) im Gehäuse 3 des Elektromotors 2 lösbar befestigbar. Beispielhaft, und nicht einschränkend, umfasst der Schnellverschluss einen Druckknopf, welcher zur Entriegelung des Statorgehäuses 10 von aussen betätigt werden kann. Alternativ kann auch eine Schnappverschluss vorgesehen sein. Damit kann der Benutzer die gesamte Statorbaugruppe in kürzester Zeit auswechseln, wenn ein elektronischer Defekt auftritt.

Die Steuerung 9 umfasst elektronische Mittel 24 zum Umschalten zwischen einem Betriebsmodus und einem Lademodus des Antriebsaggregats 1 und beispielhaft, und nicht einschränkend, eine elektrische Empfangsvorrichtung 25 zum drahtlosen Empfang von Steuerungs- und/oder Regelungssignalen. Die Steuerungs- und/oder Regelsignale werden beispielhaft, und nicht einschränkend, durch eine am Gehäuse 3 des Antriebsaggregats 1 angeordnete Bedieneinrichtung (nicht gezeichnet) drahtlos an die Steuerung 9 übertragen. Eine solche Bedieneinrichtung kann lösbar an der Aussenseite des Gehäuses des Elektromotors angeordnet sein, oder im Gehäuse eingelassen und umspritzt sein. Alternativ können die Steuerungs- und/oder Regelungssignale durch eine Fernbedienung an die Steuerung 9 übertragen werden.

Die Energieversorgungseinheit 8 umfasst zwei wiederaufladbare Akkumulatoren, welche beispielhaft Lithium-Ionen-Akkumulatoren sind. Diese Akkumulatoren sind im Statorgehäuse 10 integriert und können - nach einer Entladung im Gebrauch des Antriebsaggregats (Betriebsmodus) - im Lademodus z.B. induktiv oder im Vierquadranten-Betrieb wieder aufgeladen werden.

Für eine Aufladung via einen Vierquadranten-Betrieb eignet sich insbesondere das Verfahren mit den folgenden Schritten: a) Ankuppeln der Rotorachse 7 an eine externe rotative Antriebsvorrichtung; b) Umschalten von einem Betriebsmodus des Antriebsaggregats 1 in den Lademodus des Antriebsaggregats 1 via die Steuerung 9; c) Antreiben des Rotors 5 mittels der externen rotativen Antriebsvorrichtung bis die Energieversorgungseinheit 8 aufgeladen ist; d) Abkuppeln der Rotorachse 7 von der externen rotativen Antriebsvorrichtung; und e) Umschalten vom Lademodus in den Betriebsmodus des Antriebsaggregats 1 via die Steuerung 9.

Eine extern angetriebene Ladevorrichtung wird auf die Rotorachse 7 aufgesetzt, so dass der Elektromotor 2 des erfindungsgemässen Antriebsaggregats 1 als Generator betrieben wird und Strom erzeugt wird, um die Akkus aufzuladen. Dazu kann die Steuerung 9 einen Vierquadrantensteller umfassen. Beispielhaft umfasst dieser Vierquadrantensteller zumindest eine elektronische H-Brückenschaltung. Die H-Brückenschaltung beinhaltet insbesondere vier Transistoren. Der Vierquadrantensteller ist dazu vorgesehen eine Gleichspannung in eine Wechselspannung variabler Frequenz und variabler Pulsbreite umzuwandeln.

Obwohl wie oben beschrieben verschiedene Ausführungsformen der vorliegenden Erfindung vorliegen, sind diese so zu verstehen, dass die verschiedenen Merkmale sowohl einzeln als auch in jeder beliebigen Kombination verwendet werden können.

Diese Erfindung ist daher nicht einfach auf die oben erwähnten, besonders bevorzugten Ausführungsformen beschränkt.

## Patentansprüche

1. Antriebsaggregat (1) für ein chirurgisches Power-Tool, wobei das Antriebsaggregat umfasst:
A) einen Elektromotor (2) mit einer Längsachse (13), einem Gehäuse (3), einem Stator (4) und einem Rotor (5), wobei der Rotor (5) eine ein freies Ende (6) aufweisende Rotorachse (7) umfasst und am freien Ende (6) ein chirurgisches Werkzeug oder eine chirurgische Vorrichtung ankuppelbar ist; und
B) eine Steuerung (9) für den Elektromotor (2); wobei
C) der Stator (4) ein Statorgehäuse (10) umfasst und als Ganzes aus dem Elektromotor (2) ausbaubar, respektive in diesen einbaubar ist, wobei
D) das Antriebsaggregat (1) zusätzlich eine Energieversorgungseinheit (8) für den Elektromotor (2) und die Steuerung (9) umfasst; und
E) die Energieversorgungseinheit (8) und die Steuerung (9) im Statorgehäuse (10) angeordnet sind.

2. Antriebsaggregat nach Anspruch 1, **dadurch gekennzeichnet, dass** das Statorgehäuse (10) mittels eines Schnellverschlusses im Gehäuse (3) des Elektromotors (2) lösbar befestigbar ist.

3. Antriebsaggregat nach Anspruch 2, **dadurch gekennzeichnet, dass** der Schnellverschluss einen Druckknopf oder Schnappverschluss umfasst.

4. Antriebsaggregat nach einem der Ansprüche 1 bis 3, **dadurch gekennzeichnet, dass** das Statorgehäuse (10) ein vorderes Ende (14), ein hinteres Ende (15) und einen am hinteren Ende (15) des Statorgehäuses (10) offenen Hohlraum (16) aufweist, wobei der Hohlraum (16) entlang der Längsachse (13) hintereinander angeordnet einen ersten Längsabschnitt (17) zur Aufnahme der elektrischen Statorwicklungen (19) und einen zweiten Längsabschnitt (18) zur Aufnahme der Energieversorgungseinheit (8) und der Steuerung (9) umfasst.

5. Antriebsaggregat nach Anspruch 4, **dadurch gekennzeichnet, dass** das Statorgehäuse (10) einstückig ausgebildet ist und vorzugsweise aus Kunststoff hergestellt ist.

6. Antriebsaggregat nach Anspruch 4 oder 5, **dadurch gekennzeichnet, dass** der Hohlraum (16) des Statorgehäuses (10) mittels eines Gehäusedeckels (20) hermetisch verschliessbar ist.

7. Antriebsaggregat nach Anspruch 4 oder 5, **dadurch gekennzeichnet, dass** das Statorgehäuse (10) einen Gehäusedeckel (20) umfasst, welcher mit dem Statorgehäuse (10) vergossen oder mittels Ultraschall verschweisst ist.

8. Antriebsaggregat nach einem der Ansprüche 1 bis 7, **dadurch gekennzeichnet, dass** der Rotor (5) ein Rotorgehäuse (21) umfasst, welches eine hermetisch abgeschlossene oder abschliessbare Ummantelung (12) der elektrischen Rotorwicklungen (11) bildet.

9. Antriebsaggregat nach Anspruch 8, **dadurch gekennzeichnet, dass** das Rotorgehäuse (21) und die Rotorachse (7) einstückig ausgebildet sind.

10. Antriebsaggregat nach Anspruch 8 oder 9, **dadurch gekennzeichnet, dass** das Rotorgehäuse (21) einen Deckel (22) umfasst, welcher am Rotorgehäuse (21) befestigbar oder mit dem Rotorgehäuse (21) vergossen oder verschweisst ist.

11. Antriebsaggregat nach einem der Ansprüche 1 bis 10, **dadurch gekennzeichnet, dass** der Rotor (5) mittels Radiallagern (23) um die Längsachse (13) rotierbar im Gehäuse (3) des Elektromotors (2) gelagert ist.

12. Antriebsaggregat nach Anspruch 11, **dadurch gekennzeichnet, dass** die Radiallager (23) Wälzlager sind und am Rotorgehäuse (21) befestigt sind.

13. Antriebsaggregat nach Anspruch 11 oder 12, **dadurch gekennzeichnet, dass** die Radiallager (23) gekapselt sind.

14. Antriebsaggregat nach einem der Ansprüche 1 bis 13, **dadurch gekennzeichnet, dass** das Rotorgehäuse (21) eine zentrale Kavität (26) umfasst, welche sich entlang der Längsachse (13) erstreckt und zur Aufnahme des Stators (4) konfiguriert ist.

15. Verfahren zum Aufladen der Energieversorgungseinheit (8) eines Antriebsaggregats (1) nach einem der Ansprüche 1 bis 14, **gekennzeichnet durch** die Schritte:
a) Ankuppeln der Rotorachse (7) an eine externe rotative Antriebsvorrichtung;
b) Umschalten von einem Betriebsmodus des Antriebsaggregats (1) in einen Lademodus des Antriebsaggregats (1) via die Steuerung (9);
c) Antreiben des Rotors (5) mittels der externen rotativen Antriebsvorrichtung bis die Energieversorgungseinheit (8) aufgeladen ist;
d) Abkuppeln der Rotorachse (7) von der externen rotativen Antriebsvorrichtung; und
e) Umschalten vom Lademodus in den Betriebsmodus des Antriebsaggregats (1) via die Steuerung (9).

## Claims

1. Drive assembly (1) for a surgical power tool, the drive assembly comprising:
A) an electromotor (2) with a longitudinal axle (13), a housing (3), a stator (4) and a rotor (5), wherein the rotor (5) comprises a rotor axle (7) having a free end (6) and a surgical tool or a surgical device is couplable to the free end (6); and
B) a control (9) for the electromotor (2); wherein
C) the stator (4) comprises a stator housing (10) and can be demounted from, respectively mounted into, the electromotor (2) as a whole,
wherein
D) the drive assembly (1) additionally comprises an energy supply unit (8) for the electromotor (2) and the control (9); and
E) the energy supply unit (8) and the control (9) are arranged in the stator housing (10).

2. Drive assembly according to claim 1,
**characterised in that** the stator housing (10) is releasably fixable in the housing (3) of the electromotor (2) by means of a quick-acting closure.

3. Drive assembly according to claim 2,
**characterised in that** the quick-acting closure comprises a push button or a snap closure.

4. Drive assembly according to one of claims 1 to 3,
**characterised in that** the stator housing (10) has a front end (14), a rear end (15) and a hollow space (16) that is open at the rear end (15) of the stator housing (10), the hollow space (16) comprising, arranged one behind another along the longitudinal axle (13), a first longitudinal section (17) for receiving the electric stator windings (19) and a second longitudinal section (18) for receiving the energy supply unit (8) and the control (9).

5. Drive assembly according to claim 4,
**characterised in that** the stator housing (10) is realized integrally and is preferably made of a synthetic material.

6. Drive assembly according to claim 4 or 5,
**characterised in that** the hollow space (16) of the stator housing (10) is hermetically closable by means of a housing cover (20).

7. Drive assembly according to claim 4 or 5,
**characterised in that** the stator housing (10) comprises a housing cover (20), which is cast or ultrasound-welded with the stator housing (10).

8. Drive assembly according to one of claims 1 to 7,
**characterised in that** the rotor (5) comprises a rotor housing (21), which forms a hermetically closed or closable envelopping (12) of the electric rotor windings (11).

9. Drive assembly according to claim 8,
**characterised in that** the rotor housing (21) and the rotor axle (7) are embodied integrally.

10. Drive assembly according to claim 8 or 9,
**characterised in that** the rotor housing (21) comprises a cover (22) which is fixable at the rotor housing (21) or is cast or welded with the rotor housing (21).

11. Drive assembly according to one of claims 1 to 10,
**characterised in that** the rotor (5) is supported in the housing (3) of the electromotor (2) by radial bearings (23) such that it is rotatable around the longitudinal axle (13).

12. Drive assembly according to claim 11,
**characterised in that** the radial bearings (23) are roller bearings and are fastened on the rotor housing (21).

13. Drive assembly according to claim 11 or 12,
**characterised in that** the radial bearings (23) are encapsulated.

14. Drive assembly according to one of claims 1 to 13,
**characterised in that** the rotor housing (21) comprises a central cavity (26), which extends along the longitudinal axle (13) and is configured for receiving the stator (4).

15. Method for charging the energy supply unit (8) of a drive assembly (1) according to one of claims 1 to 14, **characterised by** the steps:
a) Coupling the rotor axle (7) to an external rotary drive device;
b) Switching from an operative mode of the drive assembly (1) into a charging mode of the drive assembly (1) via the control (9);
c) Driving the rotor (5) by means of the external rotary drive device until the energy supply unit (8) is fully charged;
d) Decoupling the rotor axle (7) from the external rotary device; and
e) Switching from the charging mode into the operative mode of the drive assembly (1) via the control (9).

## Revendications

1. Groupe propulseur (1) pour un outil électrique surgical, le groupe propulseur comprenant :
A) un moteur électrique (2) avec un arbre longitudinal (13), un boîtier (3), un stateur (4) et un roteur (5), le roteur (5) incluant un arbre de roteur (7) qui comprend une extrémité libre (6) et un outil surgical ou un dispositif surgical étant couplable à l'extrémité libre (6) ; et
B) une commande (9) pour le moteur électrique (2), où
C) le stateur (4) comporte un boîtier de stateur (10) et peut être démonté en entier du moteur électrique (2), respectivement peut être monté en entier dans le moteur électrique (2),
où
D) le groupe propulseur (1) additionnellement comprend une unité d'alimentation en énergie (8) pour le moteur électrique (2) et la commande (9) ; et
E) l'unité d'alimentation en énergie (8) et la commande (9) sont disposées dans le boîtier de stateur (10).

2. Groupe propulseur selon la revendication 1,
**caractérisé en ce que** le boîtier de stateur (10) est fixable relâchablement dans le boîtier (3) du moteur électrique (2) moyennant une fermeture rapide.

3. Groupe propulseur selon la revendication 2,
**caractérisé en ce que** la fermeture rapide comprend un bouton-poussoir ou une fermeture à cliquet.

4. Groupe propulseur selon l'une des revendications 1 à 3,
**caractérisé en ce que** le boîtier de stateur (10) comprend une extrémité avant (14), une extrémité arrière (15) et un espace creux (16) qui est ouvert à l'extrémité arrière (15) du boîtier de stateur (10),
l'espace creux (16) comprenant, disposées l'un derrière l'autre le long de l'arbre longitudinal (13), une première section longitudinale (17) pour recevoir les enroulements électriques de stateur (19) et une deuxième section longitudinale (18) pour recevoir l'unité d'alimentation en énergie (8) et la commande (9).

5. Groupe propulseur selon la revendication 4,
**caractérisé en ce que** le boîtier de stateur (10) est réalisé intégralement et est produit préférablement d'un matériau synthétique.

6. Groupe propulseur selon la revendication 4 ou 5,
**caractérisé en ce que** l'espace creux (16) du boîtier de stateur (10) peut être fermé hermétiquement par un couvercle-boîtier (20).

7. Groupe propulseur selon la revendication 4 ou 5,
**caractérisé en ce que** le boîtier de stateur (10) comprend un couvercle-boîtier (20) qui est coulé avec le boîtier de stateur (10) ou est soudé avec le boîtier de stateur (20) par ultrasons.

8. Groupe propulseur selon l'une des revendications 1 à 7,
**caractérisé en ce que** le roteur (5) comprend un boîtier de roteur (21) qui forme un enveloppe (12) des enroulements électriques de roteur (11) qui est fermé ou peut être fermé hermétiquement.

9. Groupe propulseur selon la revendication 8,
**caractérisé en ce que** le boîtier de roteur (21) et l'arbre de roteur (7) sont réalisés intégralement.

10. Groupe propulseur selon la revendication 8 ou 9,
**caractérisé en ce que** le boîtier de roteur (21) comprend un couvercle (22) qui est fixable au boîtier de roteur (21) ou est coulé ou soudé avec le boîtier de roteur (21).

11. Groupe propulseur (1) selon l'une des revendications 1 à 10,
**caractérisé en ce que** le roteur (5) est supporté dans le boîtier (3) du moteur électrique (2) par paliers radiaux (23) d'une telle manière qu'il puisse être tourné autour de l'arbre longitudinal (13).

12. Groupe propulseur selon la revendication 11,
**caractérisé en ce que** les roulements radiaux (23) sont des paliers à rouleau et sont fixés au boîtier de roteur (21).

13. Groupe propulseur selon la revendication 11 ou 12,
**caractérisé en ce que** les paliers radiaux (23) sont encapsulés.

14. Groupe propulseur selon l'une des revendications 1 à 13,
**caractérisé en ce que** le boîtier de roteur (21) comprend une cavité centrale (26) s'étendant le long de l'arbre longitudinal (13) et configurée pour recevoir le stateur (4).

15. Procédé pour charger l'unité d'alimentation en énergie (8) d'un groupe propulseur (1) selon l'une des revendications 1 à 14, **caractérisé par** les étapes :
a) Couplage de l'arbre de roteur (7) à un dispositif de propulsion rotatif externe ;
b) Commutation d'un mode operatif du groupe propulseur (1) dans un mode de charge du groupe propulseur (1) par le biais de la commande (9) ;
c) Propulsion du roteur (5) moyennant le dispositif de propulsion rotatif externe jusqu'à ce que l'unité d'alimentation en énergie soit chargée ;
d) Découplage de l'arbre de roteur (7) du dispositif de propulsion rotatif externe ; et
e) Commutation du mode de charge dans le mode opératif du groupe propulseur (1) par le biais de la commande (9).
